# EUROPEAN PATENT SPECIFICATION

(11) **EP 4 069 083 B1**
(45) Date of publication and mention of the grant of the patent: **25.10.2023**
(21) Application number: 20702099.1
(22) Date of filing: 24.01.2020
(51) Int. Cl.: A61B 5/1455, A61B 5/00, A61B 5/024

(54) **WEARABLE DEVICE AND METHOD FOR DETERMINING PHOTOPLETHYSMOGRAM**
TRAGBARE VORRICHTUNG UND VERFAHREN ZUM BESTIMMEN VON FOTOPLETHYSMOGRAM
DISPOSITIF PORTABLE ET PROCÉDÉ DE DÉTERMINATION D'UN PHOTOPLÉTHYSMOGRAMME

(43) Date of publication of application: 12.10.2022
(73) Proprietor: Huawei Technologies Co., Ltd., Shenzhen, Guangdong 518129 (CN)
(72) Inventor: ROUVALA, Markku, 16440 Kista (SE); KARKKAINEN, Asta, 16440 Kista (SE); SUHONEN, Olli, 16440 Kista (SE); BAHMANI, Nima, 16440 Kista (SE)
(74) Representative: Körber, Martin Hans
(86) International application number: PCT/EP2020/051723
(87) International publication number: WO 2021/148131

(56) References cited:
- US-A1- 2006 129 037
- US-A1- 2010 249 557
- US-A1- 2017 055 855
- US-A1- 2018 014 781
- US-A1- 2018 302 709

## Description

### TECHNICAL FIELD

The present disclosure relates generally to the field of wearable devices for health monitoring; and more specifically, to wearable devices and methods for determining a photoplethysmogram.

### BACKGROUND

Typically, photoplethysmogram is based on illuminating skin of a test subject to non-invasively analyse blood volume changes in underlying microvascular bed of tissue. Photoplethysmogram provides valuable information about cardiovascular and other physiological system, such as changes in blood flow or blood opacity associated with heart beats, breaths, blood oxygen level, and the like. Currently, there are many technical problems in conventional devices and sensing methodology used therein for determination of photoplethysmogram and for overall health monitoring. One of the primary problems is managing power consumption in such conventional devices. Typically, a light emitter is used for illumination purposes in photoplethysmography. The light emitter (e.g. a light-emitting diode) is usually the main power consumer in photoplethysmography in a conventional device. In case of a conventional wearable device that is powered by a battery having a limited size, managing power consumption in order to power such light emitter becomes even more challenging. In an example, having multiple light emitters may provide better illumination and output signal, but may also have the unwanted effect of quick drainage of the battery or may further result in an increase in size of the conventional wearable device, which is not desirable.

Another technical problem is inaccuracy in determining photoplethysmogram, which results in inaccurate health metrics, such as erroneous heart rate, breathing rate, and the like. For example, almost every person has different body features, such as different vein or artery position, in a given body portion that may be interrogated to determine photoplethysmogram. Current sensing methodology and optical setup used in conventional devices are error-prone and inadequate to handle such differences in the position of body features, and thus may not provide accurate measurements consistently for each person. In certain scenarios, at the time of determining photoplethysmogram, the test subject who may be wearing the conventional wearable device, may perform certain physical activities, such as a physical exercise, or there may be some body movements, such as movement of hands. Such movements may cause motion artefacts resulting in a much lower accuracy in the health metrics monitored using the conventional wearable device. For example, heart rate monitoring from the wrist or finger is inaccurate because of hand movement. One way to increase accuracy during physical activity or any body movements may be to feed more power to the light emitter to obtain a resultant signal with an acceptable signal quality. However, increased power consumption has the unwanted effect of draining the battery of the wearable device such that the conventional device may not have enough power to measure a test subject over the course of a day or a week even during non-exercising periods, which is not desirable.

Therefore, in light of the foregoing discussion, there exists a need to overcome the aforementioned drawbacks associated with conventional devices and methods for determining photoplethysmogram and for health monitoring.

US 2018/0302709 A1 discloses a wearable device comprising a sensor module that includes a substrate or base having an optical source and an optical detector. A housing includes a first light guide in optical communication with the optical source and a second light guide in optical communication with the optical detector. The first light guide comprises light transmissive material configured to deliver light from the optical source into a region of a body of a user at one or more predetermined locations. The second light guide comprises light transmissive material configured to collect light external to the sensor module 20 and deliver the collected light to the optical detector.

US 2010/0249557 A1 discloses a hat-based or headband sensor assembly including thin or flexible optical sensing components, such as optical fibers or ultra thin emitters or detectors.

US 2018/014781 A1 discloses an integrated window for a photosensor for use in a wearable electronic device. A photoemitter uses green light to impinge the user's skin and a photodetector is used to detect the amount of green light that is transmitted through the user's skin. In one example, a photoemitter can be positioned under a first group of fibers forming a transmit region, and a photodetector can be positioned under a second group of fibers forming a receive region.

### SUMMARY

The present disclosure seeks to provide a wearable device and a method for determining a photoplethysmogram. The present disclosure seeks to provide a solution to the existing problem of inefficient power management and an error-prone sensing methodology currently used to determine photoplethysmogram that results in inaccurate monitoring of health metrics from a conventional device. An aim of the present disclosure is to provide a solution that overcomes at least partially the problems encountered in prior art, and provides an improved wearable device and method that are able to efficiently manage power consumption and accurately determine photoplethysmogram to obtain accurate heath metrics.

The object of the present disclosure is achieved by the solutions provided in the enclosed independent claims. Advantageous implementations of the present disclosure are further defined in the dependent claims. In the following, parts of the description and drawings referring to embodiments which are not covered by the claims are not presented as embodiments of the invention, but as examples useful for understanding the invention.

In a first aspect, the present disclosure provides a wearable device for determining a photoplethysmogram. The wearable device comprises at least a first light source arranged to provide light towards at least one entry point on a proband's body when the wearable device is used by the proband. The wearable device further comprises at least first light detector arranged to detect light received from the light source through the proband's body when the wearable device is used by the proband. The wearable device further comprises a control means arranged to calculate a photoplethysmogram based on the detected light. The wearable device further comprises at least a first and a second detector optical waveguide arranged to detect light in at least two different positions on the proband's body when the wearable device is used by the proband and feed it to the at least first light detector. The first light source is a tuneable laser. The wearable device further comprises an optical waveguide including a metasurface, configured to direct different wavelengths of the tuneable laser to different entry points of the proband's body. The control means being arranged to control the wavelength of the laser directed by the optical waveguide including the metasurface to control the position of the at least one entry point.

The wearable device of the first aspect enables to obtain an improved output signal while efficiently managing power consumption. For example, an improved pulsatile physiological waveform (i.e. "AC" part of a signal), which is attributed to changes in the blood volume associated with heart beats, breaths, blood oxygen level, and the like, is obtained in the form of the output signal that results in accurate determination of the photoplethysmogram. As the first and the second detector optical waveguide are arranged to detect light in at least two different positions on the proband's body, the chances of interaction with relevant body features, such as arteries, at the proband's body is significantly increased. This improved optical setup and sensing methodology increases the number of reading (i.e. measurement) points on the proband's body covering different body features, thereby increasing accuracy in determination of the photoplethysmogram without any increase in power consumption by the single first light source.

The light source is a tuneable laser, and the optical waveguide having the metasurface can be used to direct different light bands to different locations (i.e. different entry points) to enter the proband's body. Thus, even if different people have different body features, such as different vein or artery positions, in a given body portion, the control means by use of the optical waveguide adequately handles such differences in body features (as different locations within a specific range are illuminated), and thus provides accurate measurements consistently for each person.

In a first implementation form of the first aspect, the at least first light detector is a photo diode.

As the photo diode is fed with light detected in at least two different positions on the proband's body by the first and the second detector optical waveguide, the signal quality of the output signal is significantly increased resulting in increased accuracy in determination of the photoplethysmogram.

In a second implementation form of the first aspect (which does not form part of the claimed subject-matter), the at least first light source is a light-emitting diode. The wearable device further comprises at least a first and a second source optical waveguide arranged to receive light from the at least first light source. The light of the at least first light source is guided by the at least first and the second source optical waveguide to enter the proband's body through at least two different entry points when in use.

The use of the first and a second source optical waveguide to receive light from the at least first light source, ensures power efficiency in the wearable device while further increasing accuracy in the determination of the photoplethysmogram. As light is shined on at least two different locations to enter the proband's body using multiple source optical waveguides, the chances of interaction with relevant body features, such as arteries, at the proband's body is significantly increased. This results in improved heath metrics monitoring, for example, improved clinical physiological measurements, vascular assessment, and autonomic functions. Moreover, enhanced illumination at the at least two different locations also increases the sensing capability of the first light detector, which receives light from the first and the second detector optical waveguide from at least two different positions on the proband's body.

In a third implementation form of the first aspect, the wearable device comprises one light source, and two light detectors.

By having two light detectors (e.g. multiple photodiodes), the power of the "AC" part of the output signal (i.e. quality and signal strength of the pulsatile physiological waveform of a photoplethysmography waveform) is increased without any increase in power consumption by the single light source in the wearable device.

In a fourth implementation form of the first aspect, the wearable device comprises, for each light source, at least three source optical waveguides for splitting the light from the light source.

The increase in the number of the source optical waveguides increases the likelihood of interactions with relevant body features, such as arteries, at the proband's body without any increase in power consumption by the light source. Further, increased interactions with relevant body features, such as arteries, at the proband's body increases the accuracy in determination of the photoplethysmogram, which results in accurate monitoring of heath metrics for the proband.

In a fifth implementation form of the first aspect, the wearable device comprises, for each light detector, at least three detector optical waveguides for detecting light in different positions on the proband's body.

The use of the at least three detector optical waveguides for detecting light in different positions on the proband's body, increases the number of reading (i.e. measurement) points on the proband's body covering different body features. The increase in the number of reading points increases accuracy in determination of the photoplethysmogram without any increase in power consumption by the light source.

In a sixth implementation form of the first aspect, the control means is arranged to calculate the photoplethysmogram based on the detected light from all detector optical waveguides of all light detectors.

As all detector optical waveguides of all light detectors are used to detect light, the cumulative signal strength of the output signal (e.g. the power of AC component of the output signal) is increased without any increase in power consumption by the single light source in the wearable device. This improved optical setup and sensing methodology increases the number of reading (i.e. measurement) points on the proband's body covering different body features, thereby increasing accuracy in determination of the photoplethysmogram without any increase in power consumption by the single first light source.

In seventh implementation form of the first aspect, the control means is arranged to select at least one detector optical waveguide to calculate the photoplethysmogram based on the detected light from the at least one selected detector optical waveguide.

The selection of the at least one detector optical waveguide enables to obtain an output signal having a comparatively higher signal strength (e.g. improved power of AC component of the output signal) measured from at least one point (associated with the at least one detector optical waveguide) as compared to other points on the proband's body. Thus, an accuracy in determination of the photoplethysmogram is increased without any increase in power consumption by the first light source.

In a eight implementation form of the first aspect, the control means is arranged to select at least one of the source optical waveguides to provide light to the proband's body.

The control means may localize an appropriate position of vascular features (e.g. an artery or veins) to provide light to the localized position based on the selection of at least one of the source optical waveguides. Thus, the interaction with relevant vascular features of the proband's body increases the accuracy in determination of the photoplethysmogram, which enables accurate monitoring of heath metrics for the proband.

In a ninth implementation form of the first aspect, the wearable device is a watch, a bracelet or a ring.

In conventional wearable devices, health metrics monitoring especially from the watch, the bracelet or the ring, is inaccurate because of hand movements and challenges in determining appropriate positioning of conventional sensors on skin surface. In contrast to the conventional wearable devices, the waveguide enhanced optical setup in the present wearable device improves sensing capability by increasing the number of reading (i.e. measurement) points on the proband's body covering different body features, thereby reducing any motion artefacts due to hand movements. This further increases the accuracy in determination of the photoplethysmogram without any increase in power consumption by the first light source.

In a second aspect, the present disclosure provides a method of determining a photoplethysmogram of a proband using a device. The method is implemented while the proband is wearing the device. The method comprises emitting light from a first light source towards at least one point on the proband's body. The method further comprises receiving the light by at least a first and a second detector optical waveguide arranged to detect light in at least two different positions on the proband's body, and feeding the received light to a light detector. The method further comprises calculating the photoplethysmogram based on the received light from at least one optical waveguide. The first light source is a tuneable laser. The wearable device further comprises an optical waveguide including a metasurface, and the method further comprises: using the optical waveguide including the metasurface to direct different wavelengths of the tuneable laser to different entry points of the proband's body; and controlling, by the control means, the wavelength of the tuneable laser directed by the optical waveguide including the metasurface to control the position of the at least one entry point.

The method of the second aspect achieves all the advantages and effects of the wearable device of the first aspect.

In a first implementation form of the second aspect, the method further comprises steps that are performed before the steps of the method of the second aspect, for at least a first and a second different position of the device with respect to the proband. The method further comprises emitting light from the first light source, receiving the light by the at least a first and a second detector optical waveguide, and feeding the received light to the light detector. The method further comprises calculating the photoplethysmogram; determining which one of the first and the second position provides the better result in respect to each other; and positioning the device in the position that was found to provide the better result.

As it is determined which one of the first and the second position provides the better result in respect to each other, the signal quality of the output signal is significantly increased resulting in increased accuracy in determination of the photoplethysmogram. Alternatively stated, the quality and signal strength of the pulsatile physiological waveform (i.e. AC component) of photoplethysmography waveform, is significantly increased without any increase in power consumption by the single light source in the wearable device.

It has to be noted that all devices, elements, circuitry, units and means described in the present application could be implemented in the software or hardware elements or any kind of combination thereof. All steps which are performed by the various entities described in the present application as well as the functionalities described to be performed by the various entities are intended to mean that the respective entity is adapted to or configured to perform the respective steps and functionalities. Even if, in the following description of specific embodiments, a specific functionality or step to be performed by external entities is not reflected in the description of a specific detailed element of that entity which performs that specific step or functionality, it should be clear for a skilled person that these methods and functionalities can be implemented in respective software or hardware elements, or any kind of combination thereof. It will be appreciated that features of the present disclosure are susceptible to being combined in various combinations without departing from the scope of the present disclosure as defined by the appended claims.

Additional aspects, advantages, features and objects of the present disclosure would be made apparent from the drawings and the detailed description of the illustrative implementations construed in conjunction with the appended claims that follow.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, like elements have been indicated by identical numbers.

Embodiments of the present disclosure will now be described, by way of example only, with reference to the following diagrams wherein:
FIG. 1 is a block diagram that illustrates various exemplary components of a wearable device, in accordance with an embodiment of the present disclosure;
FIG. 2 is an illustration of an exemplary scenario for implementation of a wearable device for determining photoplethysmogram, in accordance with an embodiment of the present disclosure;
FIG. 3 is an illustration that depicts different exemplary positions of vascular features in a body portion of different probands, in accordance with an embodiment of the present disclosure;
FIG. 4 is an illustration that depicts an output signal derived from a plurality of signals detected at different positions on a body portion of a proband, in accordance with an embodiment of the present disclosure;
FIG. 5 is an illustration of an exemplary wearable device with a tuneable laser as a light source and an optical waveguide, in accordance with another embodiment of the present disclosure; and
FIG. 6 is a flowchart of a method of determining a photoplethysmogram of a proband using a device, in accordance with an embodiment of the present disclosure.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

### DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

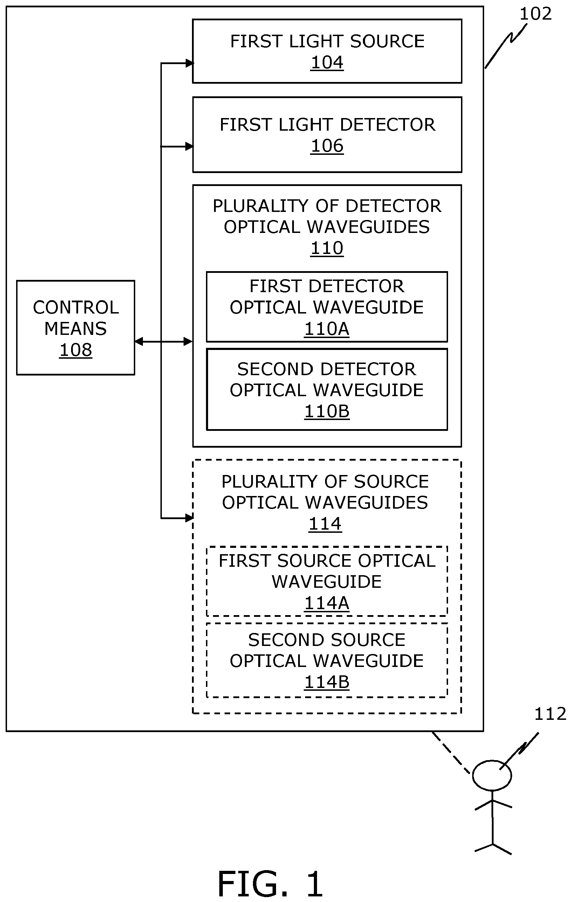

FIG. 1 is a block diagram that illustrates various exemplary components of a wearable device, in accordance with an embodiment of the present disclosure. With reference to FIG. 1, there is shown a wearable device **102.** The wearable device **102** includes a first light source **104** and one or more light detectors, such as a first light detector **106.** The wearable device **102** further includes a control means **108** and a plurality of detector optical waveguides **110,** such as first detector optical waveguide **110A** and a second detector optical waveguide **110B.** There is further shown a proband **112** associated with the wearable device **102.** Optionally, in an implementation, the wearable device **102** further includes a plurality of source optical waveguides **114,** such as a first source optical waveguide **114A** and a second source optical waveguide **114B.**

The wearable device **102** includes suitable logic, circuitry, interfaces and/or code that is configured to determine a photoplethysmogram, for example, for the proband **112** when the wearable device **102** is used by the proband **112.** The proband **112** refers to a person (e.g. a user or a given test subject) or any living creature. The photoplethysmogram associated with the proband **112** is potentially used to measure (or indicate) one or more health metrics of the proband's body (hereinafter referred to as a body of the proband **112**). Alternatively stated, the wearable device **102** is potentially used to monitor various health metrics for the body of the proband **112** who may wear the wearable device **102,** based on the determined photoplethysmogram. Example of the health metrics that is monitored by wearable device **102** includes, but is not limited to heart rate, heart rate variability (HRV), glucose level, blood pressure, and peripheral capillary oxygen saturation level (SPO₂) in the body of the proband **112.**

In accordance with an embodiment, the wearable device **102** is a watch, a bracelet or a ring. In such an embodiment, the wearable device **102** is worn by the proband **112** on a wrist or a finger. The wearable device **102** may also be worn (or attached) on an earlobe, neck, or forehead of the body of the proband **112** or any other body portion of the proband **112** that is applicable for the photoplethysmogram. Other examples of the implementation of the wearable device **102** includes, but is not limited to a tech tog, a fashion electronic device, a sports monitoring device, an identification device (e.g. specific heath metrics-based human identification), a medical device, a military device, a gaming device, or other wearable computing device.

The first light source **104** is a semiconductor device that emits light (i.e. a light emitter) when supplied with power. Alternatively stated, the first light source **104** is configured to convert electrons when powered in form of photons that are emitted from the first light source **104** in the form of electromagnetic radiation (i.e. as light). The first light source **104** is arranged to provide light towards at least one entry point on the body of the proband **112** when the wearable device **102** is used by the proband **112.** In accordance with an embodiment, at least the first light source **104** is a light-emitting diode. In an implementation, the light-emitting diode is an infrared light-emitting diode. In another implementation, the light-emitting diode is a green light-emitting diode. In yet another implementation, the light-emitting diode is a combination of an infrared light-emitting element and a green light-emitting element, which are alternatively operated in accordance with specified settings. For example, one type of coloured light-emitting element is switched 'ON' at a time to save power but have the benefit of both the green light and the infrared light wavelength in heath metrics monitoring. In accordance with another embodiment, at least the first light source **104** is a tuneable laser. The tuneable laser, when in operation, emits a wavelength of red light or near infrared light that is used for illumination of a body portion (e.g. a finger portion, a wrist portion, and the like) of the proband **112** by the wearable device **102.** Optionally, the tuneable laser is configured to emit a wavelength of green light or other wavelengths for illumination purposes.

The one or more light detectors, such as the first light detector **106,** is configured to detect an optical signal (i.e. in form of light) emitted by a light source, such as the first light source **104,** after the light propagates through a medium, such as the body of the proband **112.** The one or more light detectors act as optical receivers in the wearable device **102.** Thus, the first light detector **106** is arranged to detect light received from the at least first light source **104** through the body of the proband **112** when the wearable device **102** is used by the proband **112.** In accordance with an embodiment, at least the first light detector **106** is a photo diode. In an example, the photo diode is a positive-intrinsic-negative (PIN) diode, an avalanche photo diode, or other type of photo diodes that are able to detect light. For example, in a case where the first light source **104** is implemented as the tuneable laser in the wearable device **102,** the first light detector **106** may be implemented as the avalanche photo diode. In accordance with an embodiment, the wearable device **102** comprises one light source, such as the first light source **104,** and two light detectors, such as the first light detector **106.**

The control means **108** may include suitable logic, circuitry, interfaces and/or code that is configured to calculate the photoplethysmogram based on the light detected by one or more light detectors, such as the first light detector **106.** Examples of the control means **108** may include, but is not limited to a microprocessor, a microcontroller, a complex instruction set computing (CISC) processor, an application-specific integrated circuit (ASIC) processor, a reduced instruction set (RISC) processor, a very long instruction word (VLIW) processor, a central processing unit (CPU), a state machine, a data processing unit, and other processors or circuits. Moreover, the control means **108** may refer to one or more individual processors, processing devices, or a processing unit that is part of the wearable device **102.**

The plurality of detector optical waveguides **110,** such as the first detector optical waveguide **110A** and the second detector optical waveguide **110B,** are physical structures that are configured to guide waves, such as electromagnetic waves in the form of light. The plurality of detector optical waveguides **110,** such as the first detector optical waveguide **110A** and the second detector optical waveguide **110B,** captures light at different positions on a body portion (e.g. a finger portion) and directs (or guides) the captured light from the different positions towards a light detector, such as the first light detector **106,** with minimal loss of energy. Alternatively stated, the first detector optical waveguide **110A** and the second detector optical waveguide **110B** are arranged in the wearable device **102** to detect the light in at least two different positions on the body of the proband **112** when the wearable device **102** is used by the proband **112** and feed the detected light to the first light detector **106.** The first detector optical waveguide **110A** and the second detector optical waveguide **110B** are potentially waveguides having either a constant cross-sectional area or a variable cross-sectional area. For example, the first detector optical waveguide **110A** and the second detector optical waveguide **110B** may be waveguides having the constant cross-sectional area, such as strip waveguides, rib waveguides, and the like. In another example, the first detector optical waveguide **110A** and the second detector optical waveguide **110B** are the waveguides having the variable cross-sectional area, such as segmented waveguides, photonic crystal waveguides, and the like. In yet another example, the first detector optical waveguide **110A** and the second detector optical waveguide **110B** are laser inscribed waveguides, light pipes, optical fibres, and the like.

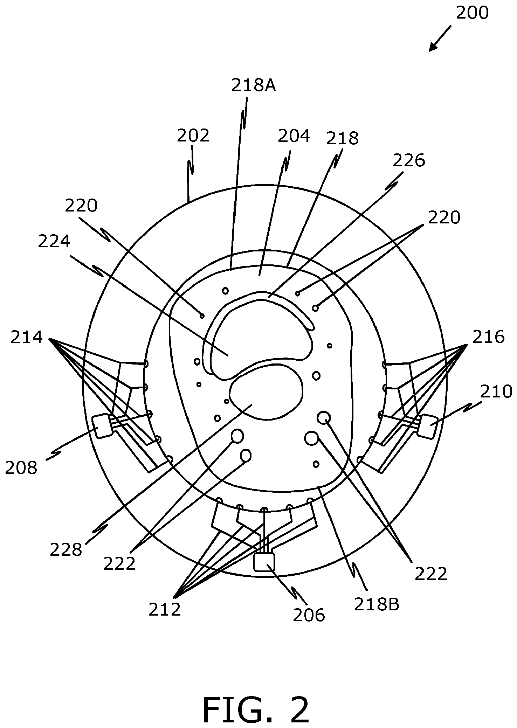

In accordance with an embodiment, the wearable device **102** further comprises at least the first and the second source optical waveguides **114A** and **114B** arranged to receive light from the at least first light source **104.** Each of the first and the second source optical waveguide **114A** and **114B** is a physical structure (e.g. an optical fibre) similar to the first detector optical waveguide **110A** and the second detector optical waveguide **110B.** In an example, each of the first and the second source optical waveguide **114A** and **114B** has a first end and a second end. The first end of each of the first and the second source optical waveguide **114A** and **114B** is coupled to the first light source **104.** The second end of each of the first and the second source optical waveguide **114A** and **114B** is arranged in the wearable device **102** such that the light emitted by the first light source **104** is guided to enter the body of the proband **112** through at least two different entry points when in use. In an example, the second end of each of the first and the second source optical waveguide **114A** and **114B** is potentially in close vicinity or in contact to the two different entry points on the body (e.g. on wrist, finger, neck, forehead, and the like) of the proband **112** when in use. In other words, the at least two different entry points are exposed to light guided through the second end of the first and the second source optical waveguide **114A** and **114B.** An exemplary arrangement of the plurality of source optical waveguides **114** is shown and described, for example, in FIG. 2. In an example, each the plurality of source optical waveguides **114** as well as the plurality of detector optical waveguides **110** is constructed using photonic components. Optionally, a core each the plurality of source optical waveguides **114** as well as the plurality of detector optical waveguides 110 is potentially deposited with a Silicon Nitride (SiₓN_{y}) material for efficient passage of light through it.

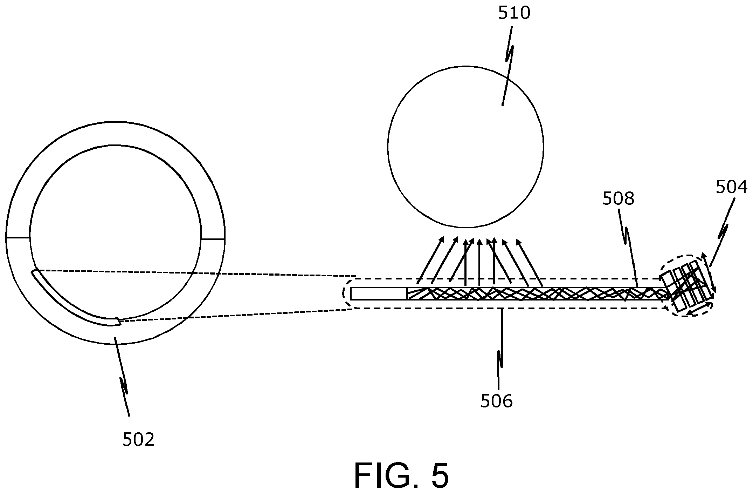

In accordance with another embodiment, the first and the second source optical waveguide **114A** and **114B** are optical waveguides with a metasurface. The first and the second source optical waveguide **114A** and **114B** are configured to guide the light emitted by the at least first light source **104** to enter the body of the proband **112** through at least two different entry points when in use. An example of the metasurface is shown and described in FIG. 5. The metasurface of the optical waveguides provides a defined surface area that allows the light to enter the body of the proband **112** through various entry points, such as two or more different entry points. Examples of implementation of the first and the second source optical waveguides **114A** and **114B** are similar to that of the first detector optical waveguide **110A** and the second detector optical waveguide **110B** as described above.

In accordance with an embodiment, the wearable device **102** comprises, for each light source, at least three source optical waveguides for splitting the light from the light source. The at least three source optical waveguides (e.g. the plurality of source optical waveguides **114**) splits and guide the light from the light source, such as the first light source **104** towards at least three entry points on the body of the proband **112.** The term "splitting" or "split" refers to receiving light from one source point and guiding the same received light to multiple destination points that resemble a separation (or dividing of) of light emitting points. In accordance with an embodiment, the wearable device **102** comprises, for each light detector, at least three detector optical waveguide detectors for detecting the light in different positions on the body of the proband **112.** The at least three detector optical waveguide detectors detects the light in at least three different positions in the body of the proband **112** and feeds it to a light detector (such as the first light detector **106**)**.**

In operation, the proband **112** may wear the wearable device **102** and power 'ON' the wearable device **102.** In an implementation, the wearable device **102** may be communicatively coupled to an external device, such as a smartphone or other display device, via a wired or a wireless communication network. In such a case, the wearable device **102** may be powered 'ON' and 'OFF' based on a user input provided by a user of the external device. In another implementation, a hardware button or a user interface may be provided in the wearable device **102** to control the wearable device 102, for example, to switch the wearable device **102,** 'ON' or 'OFF'.

The wearable device **102** includes the first light source **104** that is arranged to provide the light towards at least one entry point on the body of the proband **112** when the wearable device **102** is used by the proband **112.** The first and the second source optical waveguide **114A** and **114B** are arranged to receive the light from the at least first light source **104.** The light of the first light source **104** is guided by the at least first and the second source optical waveguide **114A** and **114B** to enter the body of the proband through at least two different entry points when in use. Optionally, for each light source, at least three source optical waveguides are arranged for splitting the light from the light source in at least three entry points on the body of the proband **112.** In cases where the first light source **104** is implemented as the tuneable laser, the control means **108** is arranged to control the wavelength of the first light source **104** to control the position of the at least one entry point (or multiple entry points) on the body of the proband **112.** Alternatively stated, one or more entry points (or locations) on the body of the proband **112** are illuminated with light from each source optical waveguides of the plurality of source optical waveguides **114.** For example, different points on skin surface of a finger, a wrist, an earlobe, neck, and the like, are illuminated with photons of light that passes through microvascular bed of tissue underlying the exposed skin surface to non-invasively analyse blood volume changes, and potentially other changes in blood, or vascular structures in exposed tissues. Moreover, as different points on skin surface are illuminated, the chances of interaction with relevant body features, such as arteries, at the body of the proband **112** is significantly increased.

The plurality of detector optical waveguides **110,** such as the first detector optical waveguide **110A** and the second detector optical waveguide **110B,** are arranged to detect the light in at least two different positions on the body of the proband **112** when the wearable device **102** is used by the proband **112** and feed it to a light detector, such as the first light detector **106.** The arrangement of at least the first detector optical waveguide **110A** and the second detector optical waveguide **110B** to detect the light in at least two different positions on the body of the proband **112** is advantageous, as it improves the sensing capability of the wearable device **102** by increasing the number of reading (i.e. measurement) points on the body of the proband **112** covering different body features, such as the arteries and veins, without any increase in power consumption by the first light source **104.** The first light detector **106** is arranged to detect the light received from the at least first light source **104** through the body of the proband **112** when the wearable device **102** is used by the proband **112.** The first light detector **106** detects the light guided by the first detector optical waveguide **110A** and the second detector optical waveguide **110B.**

The wearable device **102** that includes the control means **108** is arranged to calculate the photoplethysmogram based on the detected light from the body of the proband **112.** The accuracy of the calculation of the photoplethysmogram by the wearable device **102** depends on a number of interactions of the light with the body features (specifically the vascular features, such as arteries) in the body of the proband **112.** It is observed that greater the number of interactions, higher is the accuracy of the calculated photoplethysmogram.

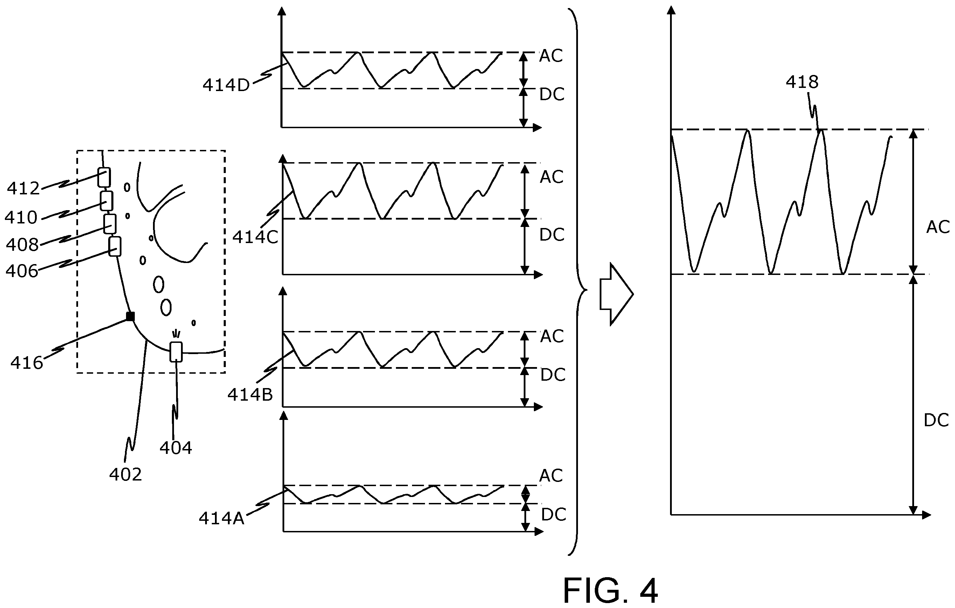

In accordance with an embodiment, the control means **108** is arranged to calculate the photoplethysmogram based on the detected light from all detector optical waveguides of all light detectors. The plurality of detector optical waveguides **110,** such as the first detector optical waveguide **110A** and the second detector optical waveguide **110B,** feeds light to respective light detectors. Generally, having multiple light detectors (e.g. the first light detector **106**) increases an overall power (or strength) of alternating current (AC) component of signals (e.g. electromagnetic waves or signals in the form of light) detected at all the light detectors. In accordance with an embodiment, the control means **108** is configured to execute a summation of such signals detected at all the light detectors to obtain a final output signal. An AC component of such summed-up signal (i.e. the final output signal) has increased power resulting in increased accuracy in determination of the photoplethysmogram without any increase in power consumption by the first light source **104.** An exemplary summation operation is shown and described, for example, in FIG. 4.

In accordance with an embodiment, the control means **108** is arranged to select at least one of the source optical waveguides (e.g. from the plurality of source optical waveguides **114**) to provide the light to the body of the proband **112.** In an example, the control means **108** selects one source optical waveguide from the three source optical waveguides for guiding the emitted light from the first light source **104.** The control means **108** potentially selects the source optical waveguide that is arranged at a specific position (e.g. an optimal location or an entry point) on the body of the proband **112** that is likely to interact with the vascular feature, such as an artery. For example, based on simulations or a test run, the control means **108** is configured to localize an appropriate position of one or more vascular features (e.g. an artery or veins) to provide light to the localized position based on the selection of at least one of the source optical waveguides of the plurality of source optical waveguides **114.** Further, such a selection potentially differs for each proband, such as the proband **112.** Thus, the control means **108** is arranged to select one or more source optical waveguides from the plurality of source optical waveguides **114** for each proband in order to increase interactions of the light with the arteries in the body of corresponding probands. The interaction with relevant vascular features of each proband increases the accuracy in determination of the photoplethysmogram, which enables accurate monitoring of heath metrics consistently for each proband.

In accordance with an embodiment, the control means **108** is arranged to select at least one detector optical waveguide to calculate the photoplethysmogram based on the detected light from the at least one selected detector optical waveguide. The control means **108** potentially selects the detector optical waveguide that captures a signal (i.e. an electromagnetic signal or wave in the form of light) having a signal strength that is greater than signal strength of signals captured by other detector optical waveguides. The selection is executed based on the interaction of the light with the arteries in the body of the proband **112.** Alternatively stated, the selection of the at least one detector optical waveguide enables to obtain an output signal having a comparatively higher signal strength (e.g. increased power of the AC component of the output signal) measured from at least one point (associated with the at least one detector optical waveguide) as compared to other points on the body of the proband **112.** Thus, an accuracy in determination of the photoplethysmogram is increased without any increase in power consumption by the first light source **104.**

FIG. 2 is an illustration of an exemplary scenario **200** for implementation of a wearable device for determining photoplethysmogram, in accordance with an embodiment of the present disclosure. FIG. 2 is described in conjunction with elements from FIG. 1. With reference to FIG. 2, there is shown the exemplary scenario **200** that includes a wearable device **202** that is worn on a finger **204.** In this embodiment, the wearable device **202** is in a form of a ring. The wearable device **202** includes a first light source **206,** a first light detector **208,** a second light detector **210,** a set of source optical waveguides **212,** a first set of detector optical waveguides **214,** and a second set of detector optical waveguides **216.** There is further shown a dorsal side **218A** and a proximal side **218B** of the finger **204** that includes an epidermis **218,** veins **220,** arteries **222,** a dorsal phalanx **224,** a tendon **226,** and a proximal phalanx **228.**

In accordance with the exemplary scenario **200,** the wearable device **202** corresponds to the wearable device **102** (FIG. 1). In this embodiment, the first light source **206** is a light-emitting diode, and each of the first light detector **208** and the second light detector **210** is a photo diode. Moreover, in this embodiment, each of the set of source optical waveguides **212,** the first set of detector optical waveguides **214,** and the second set of detector optical waveguides **216** is an optical fibre. In an example, the wearable device **202** potentially includes a battery, a memory for data storage, a network interface, which are not shown for the sake of brevity. The battery powers the first light source **206.** In an example, the wearable device **202** may be communicatively coupled (e.g. wirelessly) to an external device, such as a smartphone, via the network interface.

In accordance with the exemplary scenario **200,** the first light source **206** is arranged to provide light towards the proximal side **218B** of the finger **204,** as shown. The set of source optical waveguides **212** (e.g. five waveguides in this case) are arranged to receive light from the first light source **206** from their first end, when in operation. The light of the first light source **206** is guided by the set of source optical waveguides **212** to enter the finger **204** through five different entry points through the epidermis **218** (e.g. via the second end of each of the set of source optical waveguides **212** that are exposed towards the epidermis **218,** as shown). Thus, the epidermis **218** including the underlying tissues, such as the arteries **222,** is illuminated at five different points without any additional power consumption by the first light source **206.** The reflected light, for example, from the arteries **222,** is detected by the first set of detector optical waveguides **214** and the second set of detector optical waveguides **216.**

The first set of detector optical waveguides **214** are arranged at a defined distance from the set of source optical waveguides **212.** The first set of detector optical waveguides **214** are arranged to detect light in five different positions on the finger **204,** and feed the detected light to the first light detector **208.** Similarly, the second set of detector optical waveguides **216** are arranged at a defined distance from the set of source optical waveguides **212,** and approximately opposite to the first set of detector optical waveguides **214** for increased coverage and detection of reflected light from the arteries **222.** The second set of detector optical waveguides **216** are arranged to detect light at five different positions on the finger **204,** and to feed the detected light to the second light detector **210.** Thus, the first light detector **208** receives light (e.g. a first signal in the form of electromagnetic radiation, i.e. light) from the first set of detector optical waveguides **214** and the second light detector **210** receives light (e.g. a second signal in the form of electromagnetic radiation, i.e. light) from the second set of detector optical waveguides **216.** The control means **108** is configured to calculate a photoplethysmogram based on the detected light at the first light detector **208** and the second light detector **210.** The detected light over a period of time indicates how the reflected and scattered light intensity changes with each pulse of blood flow. The scattered light intensity usually changes in time with respect to changes in blood flow or blood opacity associated with heart beats, breaths, blood oxygen level (SPO₂), and the like. In contrast to the conventional wearable devices, the waveguide enhanced optical setup in the wearable device **202** improves sensing capability of the wearable device **202** by increasing the number of reading (i.e. measurement) points on the body of the proband **112** covering different vascular features, such as the arteries **222,** thereby reducing any motion artefacts due to hand movements, and increasing accuracy in determination of the photoplethysmogram without any increase in power consumption by the first light source **206.** Moreover, the increase in the number of reading (i.e. measurement) points on the body of the proband **112** covering different vascular features, such as the arteries **222,** enables to measure even small or otherwise conventionally undetected changes in the quantity of scattered photons that indicates varying blood flow.

In accordance with an embodiment, the control means **108** is configured to execute a summation of the signals (i.e. the first signal and the second signal) detected at the first light detector **208** and the second light detector **210** to obtain a final output signal. Typically, the reflected light detected by the first light detector **208** and the second light detector **210** is converted into the electrical signal comprising an alternating current (AC) part and a direct current (DC) part by corresponding light detectors, such as the first light detector **208** and the second light detector **210.** Thus, the photoplethysmogram waveform (i.e. the final output signal) includes a pulsatile ('AC') physiological waveform that is usually superimposed on a slowly varying ('DC') baseline. Thus, by summing up all of such signals, the quality and strength of the pulsatile physiological waveform (i.e. 'AC' component or part) of the final output signal that is attributed to, for example, cardiac synchronous changes in the blood volume with each heartbeat, is significantly increased without any increase in power consumption by the first light source **206** in the wearable device **202.** In an example, the pulsatile ('AC') physiological waveform of the output signal is potentially used for accurate measurement of accurate heart bit rates even if a proband, such as the proband **112** is performing a physical activity or hand movements. In another example, the slowly varying baseline waveform (i.e. the DC part) of the output signal may be used to measure certain other heath metrics such as respiration, sympathetic nervous system activity, and thermoregulation.

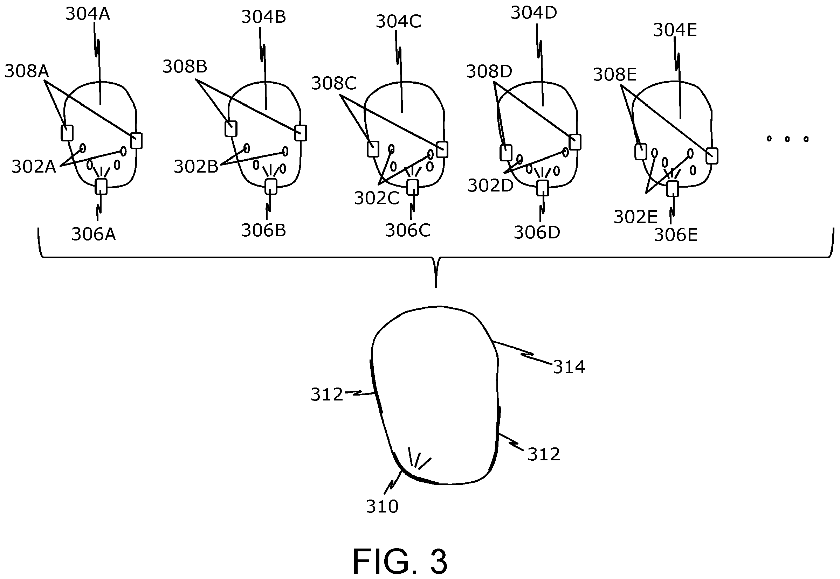

FIG. 3 is an illustration that depicts different exemplary positions of vascular features in a body portion of different probands, in accordance with an embodiment of the present disclosure. FIG. 3 is described in conjunction with elements from FIGs. 1 and 2. With reference to FIG. 3, there is shown exemplary positions of arteries **302A, 302B, 302C, 302D,** and **302E** which are different in respective finger portions **304A, 304B, 304C, 304D,** and **304E** of different probands. Accordingly, the appropriate positions of corresponding light source **306A, 306B, 306C, 306D,** and **306E** are different for different probands. Similarly, the appropriate positions of light detectors **308A, 308B, 308C, 308D,** and **308E** for respective finger portions **304A, 304B, 304C, 304D,** and **304E** of different probands are different. Alternatively stated, the positions of vascular features, such as the arteries **302A, 302B, 302C, 302D,** and **302E** in the same body portion (i.e. a finger) vary for different probands. Accordingly, the optimized positions to illuminate as well as detect light also vary for different probands. Thus, the position of a light-emitting diode **310** (i.e. a light source) for emitting light and also the positions of photodiodes **312** for capturing signals in the form of light are potentially arranged or localized in a specified range in a ring **314** (i.e. a wearable device). Such arrangement ensures enhanced coverage of different vascular features, thereby increasing accuracy in determination of the photoplethysmogram without any increase in power consumption by the light-emitting diode **310** that is powered by a battery.

FIG. 4 is an illustration that depicts an output signal derived from a plurality of signals detected at different positions on a body portion of a proband, in accordance with an embodiment of the present disclosure. FIG. 4 is described in conjunction with elements from FIGs. 1, 2, and 3. With reference to FIG. 4, there is shown a portion of a wearable device **402** having a first light source **404** and four light detectors, such as a first light detector **406,** a second light detector **408,** a third light detector 410, and a fourth light detector **412.** There is further shown an AC component of each of a first signal **414A,** a second signal **414B,** a third signal **414C,** a fourth signal **414D.** The first signal **414A,** the second signal **414B,** the third signal **414C,** and the fourth signal **414D** are detected at the first light detector **406,** the second light detector **408,** the third light detector **410,** and the fourth light detector **412,** respectively.

In accordance with an embodiment, a microprocessor **416** (e.g. an example of the control means **108** of FIG. 1) is configured to execute a summation of the first signal **414A,** the second signal **414B,** the third signal **414C,** and the fourth signal **414D** to obtain an output signal **418** having an AC component with increased power resulting in increased accuracy in determination of the photoplethysmogram by the microprocessor **416** without any increase in power consumption by the first light source **404.**

FIG. 5 is an illustration of an exemplary wearable device **502** with a tuneable laser **504** as a light source and an optical waveguide **506** with a metasurface **508,** in accordance with another embodiment of the present disclosure. FIG. 5 is described in conjunction with elements from FIGs. 1, 2, 3, and 4. With reference to FIG. 5, there is shown the wearable device **502** that includes the tuneable laser **504** as the light source. There is further shown the optical waveguide **506** with the metasurface **508** arranged at an inner side of the wearable device **502.** In this embodiment, the wearable device **502** is in the form of a ring which can be worn on a finger portion **510.** In an example, the tuneable laser **504** may be a tuneable hybrid silicon/III-V laser, known in the art. The tuneable hybrid silicon/III-V laser may include a silicon photonic integrated circuit transmitter composed of hybrid III-V/silicon lasers and silicon Mach-Zehnder modulators (MZM) operating in a specified wavelength window.

In accordance with an embodiment, the control means **512** of the wearable device **502** is configured to control a wavelength of the tuneable laser **504** to direct different light bands to different locations (i.e. different entry points towards the finger portion **510**) via the optical waveguide **506** having the metasurface **508** provided in the inner side of the wearable device **502.**

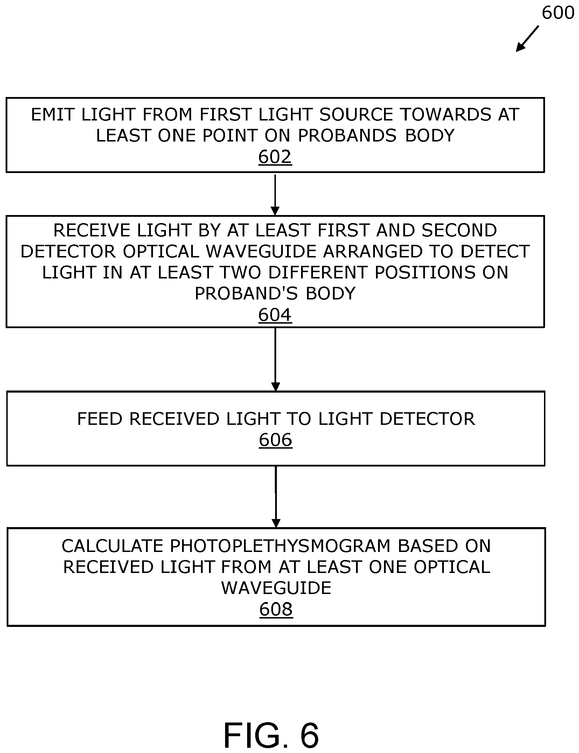

FIG. 6 is a flowchart of a method **600** of determining a photoplethysmogram of a proband using a device, in accordance with an embodiment of the present disclosure. FIG. 6 is described in conjunction with elements from FIGs. 1 to 5. The method **600** is executed by a wearable device **102, 202, 402,** or **502** described, for example, in Figs. 1 to 5. The method **600** includes steps **602** and **608.**

At step **602,** light is emitted from a first light source towards at least one point on the proband's body (such as the proband **112**)**.** Examples of the first light source are the first light source **104, 206,** or **404,** the light sources **306A, 306B, 306C, 306D,** or **306E,** or the tuneable laser **504,** shown and described, for example, in FIGs. 1 to 5.

At step **604,** the light is received by at least a first and a second detector optical waveguide arranged to detect light in at least two different positions on the proband's body. Examples of the first and the second detector optical waveguide are the plurality of detector optical waveguides **110** (such as the first detector optical waveguide **110A** and the second detector optical waveguide **110B**), the first set of detector optical waveguides **214,** the second set of detector optical waveguides **216,** or the optical waveguide **506,** shown and described, for example, in FIGs. 1, 2, and 5.

At step **606,** the received light is fed to a light detector. Examples of the light detector are the first light detector **106, 208,** or **406,** a second light detector **210** or **408,** the light detectors **308A, 308B, 308C, 308D,** and **308E,** the third light detector **410,** or the fourth light detector **412,** shown and described, for example, in FIGs. 1 to 5.

At step **608,** a photoplethysmogram is calculated based on the received light from at least one optical waveguide. In an example, the photoplethysmogram is calculated based on the light received at the plurality of detector optical waveguides **110.** The control means **108** (FIG. 1) is arranged to calculate the photoplethysmogram based on the detected light from the body of the proband **112.**

In accordance with an embodiment, the method **600** further comprises performing certain operations or steps before the steps **602** to **608** for at least a first and a second different position of the device (such wearable device **102, 202, 402,** or **502**) with respect to the proband **112.** Such operations or steps include emitting light from the first light source **104, 206,** or **404;** receiving the light by the at least the first detector optical waveguide **110A** and the second detector optical waveguide **110B,** and feeding the received light to the light detector (such as first light detector **106, 208,** or **406**)**.** The method **600** further includes calculating the photoplethysmogram and determining which one of the first and the second position provides a better result with respect to each other. For example, the control means **108** is configured to perform a test run (or a simulation or an experimentation) to localize an appropriate position of vascular features (e.g. an artery or veins) to provide light to the localized position for the first and the second different position of the device (such wearable device **102, 202, 402,** or **502**) with respect to the proband **112.** The better result refers to an accuracy level of the measurements of health metrics based on the calculated photoplethysmogram for the first and the second different position of the device (such wearable device **102, 202, 402,** or **502**)**.** The method 600 further includes positioning the device in the position that was found to provide the better result. As it is determined which one of the first and the second position provides the better result in respect to each other, the device is positioned accordingly. Thus, the signal quality of the output signal is significantly increased resulting in increased accuracy in determination of the photoplethysmogram. Alternatively stated, the quality and signal strength of the pulsatile physiological waveform (i.e. AC component) of the output signal (such as the output signal **418**) is significantly increased without any increase in power consumption by the first light source **104, 206,** or **404.**

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural. The word "exemplary" is used herein to mean "serving as an example, instance or illustration". Any embodiment described as "exemplary" is not necessarily to be construed as preferred or advantageous over other embodiments and/or to exclude the incorporation of features from other embodiments. The word "optionally" is used herein to mean "is provided in some embodiments and not provided in other embodiments". It is appreciated that certain features of the present disclosure, which are, for clarity, described in the context of separate embodiments, may also be provided in combination in a single embodiment. Conversely, various features of the invention, which are, for brevity, described in the context of a single embodiment, may also be provided separately or in any suitable combination or as suitable in any other described embodiment of the disclosure.

## Claims

1. A wearable device (102, 202, 402, 502) for determining a photoplethysmogram, comprising
at least a first light source (104, 206, 404) arranged to provide light towards at least one entry point on a proband's body when the wearable device (102, 202, 402, 502) is used by the proband (112); and
at least a first light detector (106, 208, 406) arranged to detect light received from the at least first light source (104, 206, 404) through the proband's body when the wearable device (102, 202, 402, 502) is used by the proband (112); and
a control means (108) arranged to calculate a photoplethysmogram based on the detected light; and
at least a first and a second detector optical waveguide (110A, 110B) arranged to detect light in at least two different positions on the proband's body when the wearable device (102, 202, 402, 502) is used by the proband (112) and feed it to the at least first light detector (106, 208, 406);
**characterized in that**:
the at least first light source (104, 206, 404) is a tuneable laser (504), wherein the wearable device (102, 202, 402, 502) further comprises an optical waveguide (506) including a metasurface (508), wherein the optical waveguide (506) including the metasurface (508) is configured to direct different wavelengths of the tuneable laser (504) to different entry points of the proband's body, and wherein the control means (108) is arranged to control the wavelength of the tuneable laser (504) directed by the optical waveguide (506) including the metasurface (508) to control the position of the at least one entry point.

2. A wearable device (102, 202, 402, 502) according to claim 1, wherein the at least first light detector (106, 208, 406) is a photo diode.

3. A wearable device (102, 202, 402, 502) according to claim 1 comprising one light source, and two light detectors.

4. A wearable device (102, 202, 402, 502) according to claim 1 comprising, for each light source, at least three source optical waveguides for splitting the light from the light source.

5. A wearable device (102, 202, 402, 502) according to any one of the claims 3 and 4, comprising, for each light detector, at least three detector optical waveguides for detecting light in different positions on the proband's body.

6. A wearable device (102, 202, 402, 502) according to any one of the preceding claims, wherein the control means (108) is arranged to calculate the photoplethysmogram based on the detected light from all detector optical waveguides of all light detectors.

7. A wearable device (102, 202, 402, 502) according to any one of the claims 1 - 6, wherein the control means (108) is arranged to select at least one detector optical waveguide to calculate the photoplethysmogram based on the detected light from the at least one selected detector optical waveguide.

8. A wearable device (102, 202, 402, 502) according to any one of the preceding claims, which is a watch, a bracelet or a ring (314).

9. A method (600) of determining a photoplethysmogram of a proband (112) using a device according to any one of the claims 1 - 8, comprising the steps, while the proband (112) is wearing the device, of
emitting light from a first light source (104, 206, 404) towards at least one point on the proband's body;
receiving the light by at least a first and a second detector optical waveguide (110A, 110B) arranged to detect light in at least two different positions on the proband's body and feeding the received light to a light detector; and
calculating, by a control means (108), the photoplethysmogram based on the received light from at least one optical waveguide;
wherein the at least first light source (104, 206, 404) is a tuneable laser (504), wherein the wearable device (102, 202, 402, 502) further comprises an optical waveguide (506) including a metasurface (508), and the method further comprises: using the optical waveguide (506) including the metasurface (508) to direct different wavelengths of the tuneable laser (504) to different entry points of the proband's body; and controlling, by the control means (108), the wavelength of the tuneable laser (504) directed by the optical waveguide (506) including the metasurface (508) to control the position of the at least one entry point.

10. A method (600) according to claim 9, wherein, before the steps of claim 9, the following steps are performed for at least a first and a second different position of the device with respect to the proband (112):
emitting light from the first light source (104, 206, 404);
receiving the light by the at least a first and a second detector optical waveguide (110A, 110B) and feeding the received light to the light detector;
calculating the photoplethysmogram;
determining which one of the first and the second position provides a better result with respect to each other; and
positioning the device in the position that was found to provide the better result.

## Patentansprüche

1. Tragbare Vorrichtung (102, 202, 402, 502) zum Bestimmen eines Photoplethysmogramms, umfassend: mindestens eine erste Lichtquelle (104, 206, 404), die dazu ausgelegt ist, Licht in Richtung auf mindestens einen Eintrittspunkt an dem Körper eines Probanden bereitzustellen, wenn die tragbare Vorrichtung (102, 202, 402, 502) von dem Probanden (112) verwendet wird; und mindestens einen ersten Lichtdetektor (106, 208, 406), der dazu ausgelegt ist, Licht zu detektieren, das von der mindestens ersten Lichtquelle (104, 206, 404) durch den Körper des Probanden empfangen wird, wenn die tragbare Vorrichtung (102, 202, 402, 502) von dem Probanden (112) verwendet wird; und eine Steuerungseinrichtung (108), die dazu ausgelegt ist, auf der Grundlage des detektierten Lichts ein Photoplethysmogramm zu berechnen; und
mindestens einen ersten und einen zweiten optischen Detektorwellenleiter (110A, 110B), die dazu ausgelegt sind, Licht an mindestens zwei verschiedenen Positionen an dem Körper des Probanden zu detektieren, wenn die tragbare Vorrichtung (102, 202, 402, 502) von dem Probanden (112) verwendet wird, und es dem mindestens ersten Lichtdetektor (106, 208, 406) zuzuführen;
**dadurch gekennzeichnet, dass**:
die mindestens erste Lichtquelle (104, 206, 404) ein abstimmbarer Laser (504) ist, wobei die tragbare Vorrichtung (102, 202, 402, 502) ferner einen optischen Wellenleiter (506) umfasst, der eine Metaoberfläche (508) beinhaltet, wobei der optische Wellenleiter (506), der die Metaoberfläche (508) beinhaltet, dazu konfiguriert ist, verschiedene Wellenlängen des abstimmbaren Lasers (504) auf verschiedene Eintrittspunkte des Körpers des Probanden zu richten, und wobei die Steuerungseinrichtung (108) dazu ausgelegt ist, die Wellenlänge des abstimmbaren Lasers (504) zu steuern, die durch den optischen Wellenleiter (506), der die Metaoberfläche (508) beinhaltet, gerichtet wird, um die Position des mindestens einen Eintrittspunkts zu steuern.

2. Tragbare Vorrichtung (102, 202, 402, 502) nach Anspruch 1, wobei der mindestens erste Lichtdetektor (106, 208, 406) eine Photodiode ist.

3. Tragbare Vorrichtung (102, 202, 402, 502) nach Anspruch 1, umfassend eine Lichtquelle und zwei Lichtdetektoren.

4. Tragbare Vorrichtung (102, 202, 402, 502) nach Anspruch 1, umfassend, für jede Lichtquelle, mindestens drei optische Quellenwellenleiter zum Aufteilen des Lichts von der Lichtquelle.

5. Tragbare Vorrichtung (102, 202, 402, 502) nach einem der Ansprüche 3 und 4, umfassend, für jeden Lichtdetektor, mindestens drei optische Detektorwellenleiter zum Detektieren von Licht an verschiedenen Positionen an dem Körper des Probanden.

6. Tragbare Vorrichtung (102, 202, 402, 502) nach einem der vorhergehenden Ansprüche, wobei die Steuerungseinrichtung (108) dazu ausgelegt ist, das Photoplethysmogramm auf der Grundlage des detektierten Lichts von allen optischen Detektorwellenleitern aller Lichtdetektoren zu berechnen.

7. Tragbare Vorrichtung (102, 202, 402, 502) nach einem der Ansprüche 1-6, wobei die Steuerungseinrichtung (108) dazu ausgelegt ist, mindestens einen optischen Detektorwellenleiter auszuwählen, um das Photoplethysmogramm auf der Grundlage des detektierten Lichts von dem mindestens einen ausgewählten optischen Detektorwellenleiter zu berechnen.

8. Tragbare Vorrichtung (102, 202, 402, 502) nach einem der vorhergehenden Ansprüche, die eine Uhr, ein Armband oder ein Ring (314) ist.

9. Verfahren (600) zum Bestimmen eines Photoplethysmogramms eines Probanden (112) unter Verwendung einer Vorrichtung nach einem der Ansprüche 1-8, umfassend die folgenden Schritte, während der Proband (112) die Vorrichtung trägt: Aussenden von Licht, von einer ersten Lichtquelle (104, 206, 404) in Richtung auf mindestens einen Punkt an dem Körper des Probanden;
Empfangen des Lichts durch mindestens einen ersten und einen zweiten optischen Detektorwellenleiter (110A, 110B), die dazu ausgelegt sind, Licht an mindestens zwei verschiedenen Positionen an dem Körper des Probanden zu detektieren, und Zuführen des empfangenen Lichts zu einem Lichtdetektor; und Berechnen, durch eine Steuerungseinrichtung (108), des Photoplethysmogramms auf der Grundlage des empfangenen Lichts von mindestens einem optischen Wellenleiter;
wobei die mindestens erste Lichtquelle (104, 206, 404) ein abstimmbarer Laser (504) ist, wobei die tragbare Vorrichtung (102, 202, 402, 502) ferner einen optischen Wellenleiter (506) umfasst, der eine Metaoberfläche (508) beinhaltet und das Verfahren ferner Folgendes umfasst: Verwenden des optischen Wellenleiters (506), der die Metaoberfläche (508) beinhaltet, um verschiedene Wellenlängen des abstimmbaren Lasers (504) auf verschiedene Eintrittspunkte des Körpers des Probanden zu richten; und Steuern, durch die Steuerungseinrichtung (108), der Wellenlänge des abstimmbaren Lasers (504), die durch den optischen Wellenleiter (506), der die Metaoberfläche (508) beinhaltet, gerichtet wird, um die Position des mindestens einen Eintrittspunkts zu steuern.

10. Verfahren (600) nach Anspruch 9, wobei vor den Schritten von Anspruch 9 die folgenden Schritte für mindestens eine erste und eine zweite verschiedene Position der Vorrichtung in Bezug auf den Probanden (112) durchgeführt werden:
Aussenden von Licht von der ersten Lichtquelle (104, 206, 404); Empfangen des Lichts durch den mindestens einen ersten und einen zweiten optischen Detektorwellenleiter (110A, 110B) und Zuführen des empfangenen Lichts zu dem Lichtdetektor;
Berechnen des Photoplethysmogramms;
Bestimmen, welche von der ersten und der zweiten Position im Verhältnis zueinander ein besseres Ergebnis bereitstellt; und Positionieren der Vorrichtung an der Position, für die festgestellt wurde, dass sie das bessere Ergebnis bereitstellt.

## Revendications

1. Dispositif portable (102, 202, 402, 502) de détermination d'un photopléthysmogramme, comprenant au moins une première source lumineuse (104, 206, 404) agencée pour fournir de la lumière vers au moins un point d'entrée sur le corps d'un proposant lorsque le dispositif portable (102, 202, 402, 502) est utilisé par le proposant (112) ; et
au moins un premier détecteur de lumière (106, 208, 406) agencé pour détecter la lumière reçue de l'au moins première source lumineuse (104, 206, 404) à travers le corps du proposant lorsque le dispositif portable (102, 202, 402, 502) est utilisé par le proposant (112) ; et
un moyen de commande (108) agencé pour calculer un photopléthysmogramme sur la base de la lumière détectée ; et au moins un premier et un second guide d'onde optique détecteur (110A, 110B) agencés pour détecter la lumière dans au moins deux positions différentes sur le corps du proposant lorsque le dispositif portable (102, 202, 402, 502) est utilisé par le proposant (112) et l'acheminer vers au moins le premier détecteur de lumière (106, 208, 406) ;
**caractérisé en ce que** :
l'au moins première source lumineuse (104, 206, 404) est un laser accordable (504), dans lequel le dispositif portable (102, 202, 402, 502) comprend en outre un guide d'ondes optique (506) comportant une métasurface (508), dans lequel le guide d'ondes optique (506) comportant la métasurface (508) est configuré pour diriger différentes longueurs d'onde du laser accordable (504) vers différents points d'entrée du corps du proposant, et dans lequel le moyen de commande (108) est agencé pour commander la longueur d'onde du laser accordable (504) dirigé par le guide d'ondes optique (506) comportant la métasurface (508) pour commander la position de l'au moins un point d'entrée.

2. Dispositif portable (102, 202, 402, 502) selon la revendication 1, dans lequel l'au moins premier détecteur de lumière (106, 208, 406) est une photodiode.

3. Dispositif portable (102, 202, 402, 502) selon la revendication 1, comprenant une source lumineuse et deux détecteurs de lumière.

4. Dispositif portable (102, 202, 402, 502) selon la revendication 1 comprenant, pour chaque source lumineuse, au moins trois guides d'ondes optiques sources pour séparer la lumière de la source lumineuse.

5. Dispositif portable (102, 202, 402, 502) selon l'une quelconque des revendications 3 et 4, comprenant, pour chaque détecteur de lumière, au moins trois guides d'ondes optiques détecteurs pour détecter la lumière dans différentes positions sur le corps du proposant.

6. Dispositif portable (102, 202, 402, 502) selon l'une quelconque des revendications précédentes, dans lequel le moyen de commande (108) est agencé pour calculer le photopléthysmogramme sur la base de la lumière détectée à partir de tous les guides d'ondes optiques détecteurs de tous les détecteurs de lumière.

7. Dispositif portable (102, 202, 402, 502) selon l'une quelconque des revendications 1 à 6, dans lequel le moyen de commande (108) est agencé pour sélectionner au moins un guide d'ondes optique détecteur pour calculer le photopléthysmogramme sur la base de la lumière détectée provenant de l'au moins un guide d'ondes optique détecteur sélectionné.

8. Dispositif portable (102, 202, 402, 502) selon l'une quelconque des revendications précédentes, qui est une montre, un bracelet ou une bague (314).

9. Procédé (600) de détermination d'un photopléthysmogramme d'un proposant (112) à l'aide d'un dispositif selon l'une quelconque des revendications 1 à 8, comprenant les étapes, pendant que le proposant (112) porte le dispositif, d'émission de la lumière provenant d'une première source lumineuse (104, 206, 404) vers au moins un point sur le corps du proposant ;
de réception de la lumière par au moins un premier et un second guide d'onde optique détecteur (110A, 110B) agencés pour détecter la lumière dans au moins deux positions différentes sur le corps du proposant et l'alimentation de la lumière reçue vers un détecteur de lumière ; et
de calcul, par un moyen de commande (108), du photopléthysmogramme sur la base de la lumière reçue provenant d'au moins un guide d'onde optique ;
dans lequel l'au moins première source lumineuse (104, 206, 404) est un laser accordable (504), dans lequel le dispositif portable (102, 202, 402, 502) comprend en outre un guide d'ondes optique (506) comportant une métasurface (508), et le procédé comprend en outre : l'utilisation du guide d'ondes optiques (506) comportant la métasurface (508) pour diriger différentes longueurs d'onde du laser accordable (504) vers différents points d'entrée du corps du proposant ; et la commande, par le moyen de commande (108), de la longueur d'onde du laser accordable (504) dirigé par le guide d'ondes optique (506) comportant la métasurface (508) pour commander la position de l'au moins un point d'entrée.

10. Procédé (600) selon la revendication 9, dans lequel, avant les étapes de la revendication 9, les étapes suivantes sont exécutées pour au moins une première et une seconde position différente du dispositif par rapport au proposant (112) :
l'émission de lumière provenant de la première source lumineuse (104, 206, 404) ;
la réception de la lumière par l'au moins un premier et un second guide d'onde optique détecteur (110A, 110B) et l'alimentation de la lumière reçue vers le détecteur de lumière ;
le calcul du photopléthysmogramme ;
le fait de déterminer laquelle parmi la première et la seconde position fournit un meilleur résultat l'une par rapport à l'autre ; et
le positionnement de l'appareil dans la position qui s'est avérée fournir le meilleur résultat.
